# EUROPEAN PATENT SPECIFICATION

(11) **EP 2 945 948 B1**
(45) Date of publication and mention of the grant of the patent: **29.04.2020**
(21) Application number: 14700674.6
(22) Date of filing: 16.01.2014
(51) Int. Cl.: C07D 487/04, A61P 7/02

(54) **CRYSTALLINE FORM II OF ANAGRELIDE HYDROCHLORIDE MONOHYDRATE**
KRISTALLINE FORM II VON ANAGRELIDHYDROCHLORIDMONOHYDRAT
FORME CRISTALLINE II DE CHLORHYDRATE D'ANAGRÉLIDE MONOHYDRATÉ

(30) Priority: 18.01.2013 WO PCT/EP2013/050934; 18.01.2013 EP 13151863
(43) Date of publication of application: 25.11.2015
(73) Proprietor: Synthon BV, 6545 CM Nijmegen (NL)
(72) Inventor: WESTHEIM, Raymond Jozef Hubertus, NL-6545 CM Nijmegen (NL)
(74) Representative: Mendivil Gil, Maria Dolores
(86) International application number: PCT/EP2014/050759
(87) International publication number: WO 2014/111444

(56) References cited:
- WO-A1-2009/087673
- US-A- 3 932 407
- US-A- 4 146 718
- US-A- 5 391 737
- Igor Ivanisevic ET AL: "Uses of X-Ray Powder Diffraction In the Pharmaceutical Industry", Pharmaceutical Sciences Encyclopedia: Drug Discovery, Development, and Manufacturing, 25 June 2010 (2010-06-25), pages 1-42, XP055007590, DOI: 10.1002/9780470571224.pse414 Retrieved from the Internet: URL:http://onlinelibrary.wiley.com/store/1 0.1002/9780470571224.pse414/asset/pse414.p df?v=1&t=gsssyq3r&s=316303db60793cc15e15c8 7a2d3b6ec61b51414e [retrieved on 2011-09-20]

## Description

### BACKGROUND OF THE INVENTION

The present invention relates to crystalline form II of anagrelide hydrochloride monohydrate.

Anagrelide is a pharmaceutically active compound useful for the treatment of (essential) thrombocythaemia. It is represented by formula (I).

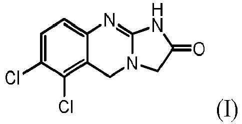

The commercially marketed products Xagrid® and Agrylin® contain the hydrochloride monohydrate salt of the compound (I). Anagrelide hydrochloride monohydrate is an off-white powder that is very slightly soluble in water.

Anagrelide was disclosed in US 3932407. Preparation of crystalline anagrelide hydrochloride is described in Example 25. No melting point, IR or XRPD data are disclosed. In this patent, acetonitrile/ethereal HCl is used as solvent/salt forming acid system to produce anagrelide hydrochloride from anagrelide free base. The use of ether is not suitable for large scale production due to the risk for explosion. Acetonitrile is not preferred as it is a less acceptable class 2 solvent.

US 4146718 teaches in Example 4 the preparation of crystalline anagrelide hydrochloride monohydrate from anagrelide base in boiling methanol by adding concentrated HCl. Precipitation was effected by adding ether as an anti-solvent. According to a comparative example, anagrelide hydrochloride was prepared by means of recrystallisation of anagrelide hydrochloride from an ethanolic HCl solution. There is no reference as to which crystalline form is obtained.

US 5391737 discloses a process to make anagrelide hydrochloride semihydrate. It makes use of methanol as the solvent and ethanolic HCl as the salt forming agent. This process is not desired as methanol is a pharmaceutically less acceptable solvent. Furthermore, the reaction is performed at reflux temperature.

In Example 6 of WO 2009/087673, a preparation route for crystalline anagrelide hydrochloride monohydrate was disclosed using concentrated HCl as the salt forming agent and ethanol as the solvent. Furthermore, the salt preparation was performed at reflux temperature.

As anagrelide hydrochloride monohydrate is an important pharmaceutical, any improvement in the state of the art relevant to this salt, particularly with the aim to identify stable crystalline forms with improved physical properties, is needed.

### BRIEF DESCRIPTION OF THE DRAWINGS

Figure 1: XRPD pattern of form I of anagrelide hydrochloride monohydrate
Figure 2: XRPD pattern of form II of anagrelide hydrochloride monohydrate
Figure 3: XRPD pattern of form III of anagrelide hydrochloride monohydrate
Figure 4: Overlay of the XRPD patterns of anagrelide hydrochloride monohydrate forms I to III
Figure 5: Solubility curves of form II of anagrelide hydrochloride monohydrate compared with form I

### DETAILED DESCRIPTION OF THE INVENTION

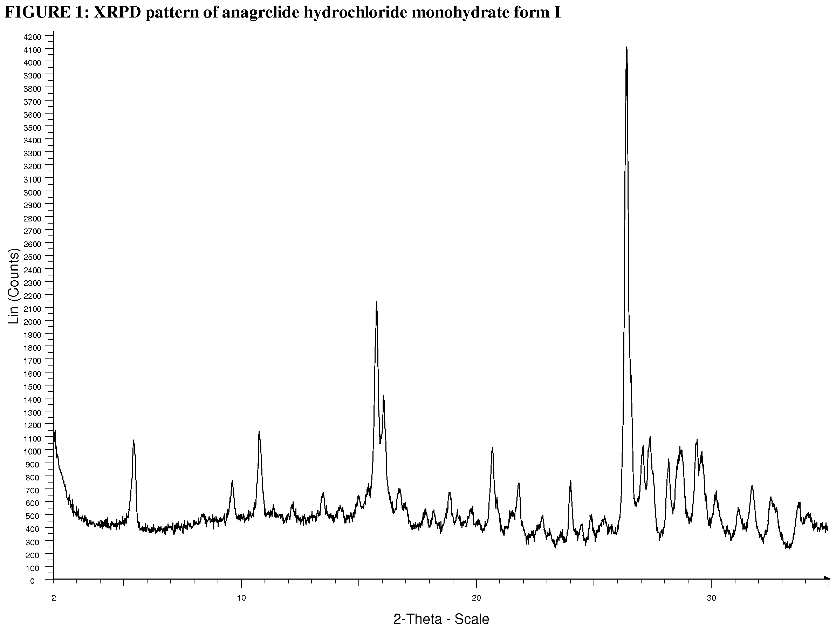

The present invention is based on the discovery of polymorphism and the identification of a specific useful polymorphic form of anagrelide hydrochloride monohydrate for incorporation into a pharmaceutical composition. After repeating the prior art processes for making anagrelide hydrochloride monohydrate in solid state as indicated in the comparative examples, it was found that these processes yielded a certain crystalline form of anagrelide hydrochloride monohydrate, herein denoted as form I. The crystalline anagrelide hydrochloride monohydrate form I can generally be characterized by an XRPD pattern as shown in Figure 1, and has characteristic diffraction peaks at the following angles: 5.4°, 9.6°, 13.4°, 15.8°, 16.1°, 16.7°, 17.0°, 17.8°, 18.2°, 19.2°, 20.7°, 24.0°, 26.4°, 28.2°, and 31.8° degrees 2 theta (± 0.2 degrees 2 theta). For clarity, whenever the expression "± 0.2 degrees 2 theta" is used herein it refers to the variance for each peak angle listed.

In the present invention, a particularly useful crystalline polymorph of anagrelide hydrochloride monohydrate was discovered. This polymorph has a crystal structure different from form I. This polymorph is characterized by a distinctly different X-ray powder diffraction (XRPD) pattern comprising signals at diffraction angles at 14.2°, 15.3°, 16.3°, 18.7°, 19.5°, 22.4°, 23.1°, 23.5°, 23.7°, 24.2°, 26.6°, and 33.6° degrees 2 theta (± 0.2 degrees 2 theta), at which angles form I shows no or just tiny peaks.

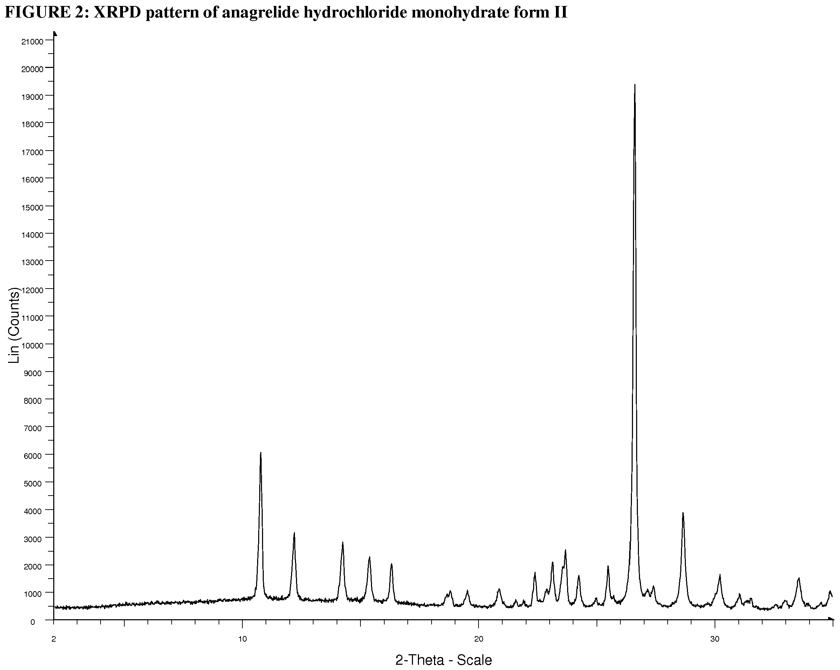

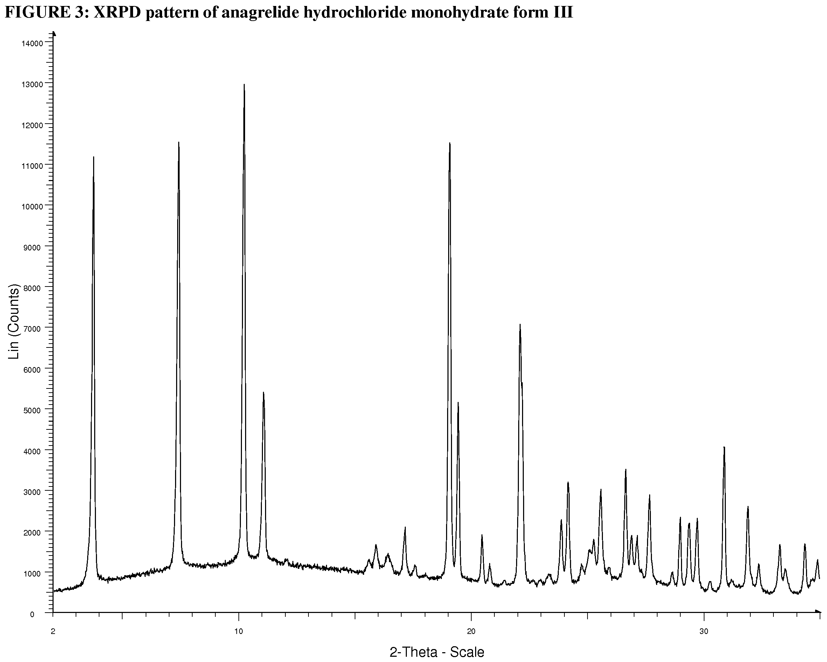

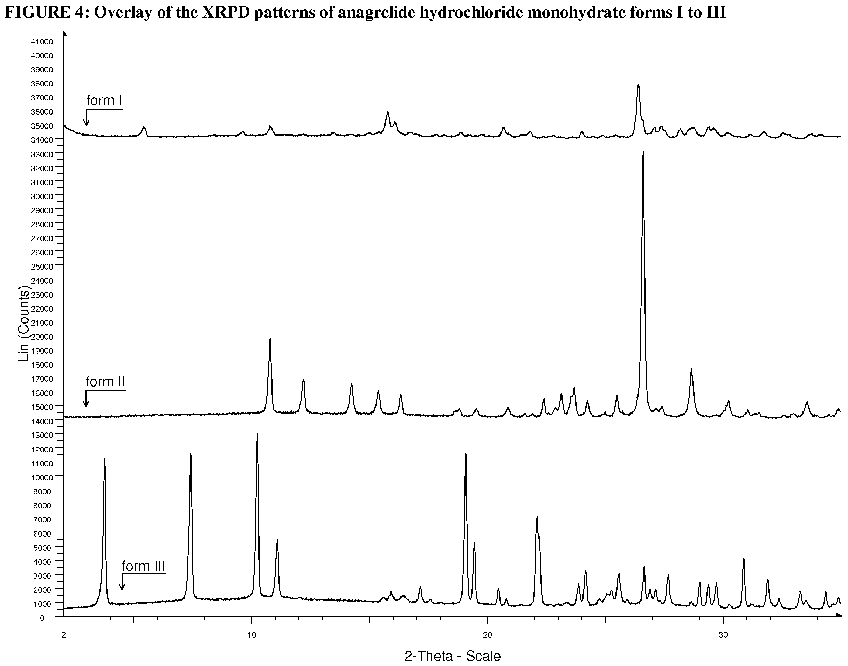

Typically, the XRPD pattern of the new polymorph substantially corresponds to that as shown in Figure 2. "Substantially corresponds" is meant to cover variations/differences in the pattern that would be understood by a worker skilled in the art not to represent a difference in the crystal structure, but rather differences in the technique, equipment, sample preparation, impurities, etc. Differences in a pattern or spectra that are not attributable to these factors indicate that the pattern in question does not "substantially correspond" to the reference pattern. For clarity, the XRPD pattern of Figure 1 does not substantially correspond to that of Figure 2 or Figure 3 as can be clearly seen in the overlay in Figure 4. Conversely, a pattern or spectrum may "substantially correspond" to e.g., Figure 2, even though it is not an identical/superimposable image. Further, the XRPD pattern of an actual sample of anagrelide hydrochloride monohydrate may differ from the pattern as shown in any one of Figures 1 to 4 with respect to intensities of the respective peaks due to preferred orientation of the crystal lattices in the powder sample.

This new polymorph of anagrelide hydrochloride monohydrate of the above XRPD pattern shall be denoted in this application as "anagrelide hydrochloride monohydrate form II," or simply "form II".

The present invention includes anagrelide hydrochloride monohydrate Form II as an isolated species (i.e. after removal of the residual solvents used in the method of making it), especially in an essentially (chemically) pure form and/or essentially polymorphically pure form. "Essentially pure" refers to at least 70% pure, preferably at least 80% pure, more preferably at least 90% pure, and still more preferably at least 95%, including at least 98% pure, at least 99% pure, and at least 99.8% pure, with respect to the presence of anagrelide hydrochloride monohydrate in a sample. "Essentially polymorphically pure" refers to at least 90% pure, preferably at least 95%, including at least 98% pure, at least 99% pure, and at least 99.8% pure, with respect to the presence of form II in a sample. Typically in order for a sample of anagrelide hydrochloride monohydrate to have an XRPD pattern that substantially corresponds to Figure 2, the Form II sample must be in essentially (chemically) pure form (typically at least 90% pure) and essentially polymorphically pure form (typically at least 90% polymorphically pure).

The form II of the present invention is polymorphically stable at ambient temperature and even more stable than form I, at least in solvents as ethanol, 2-propanol, acetone, and ethyl acetate as shown in the examples.

Form II is also more stable against aqueous hydrolysis than form I as shown in the stability experiments in which both crystal forms were stirred in demi-water for up to 180 minutes.

Surprisingly, besides being more polymorphically stable than form I, at room temperature form II is also more soluble in USP acetate buffer pH 4.5, USP phosphate buffer pH 6.8, Milli-Q water and 0.1 M HCl than form I. One possible explanation for this higher solubility of the more stable form II could be a better wettability. As a result, form II has at least the following advantages over form I:
1) Better thermodynamic stability at temperatures below 70°C;
2) Better stability against aqueous hydrolysis; and
3) Higher solubility, despite thermodynamic stability.

Accordingly, the form II of anagrelide hydrochloride monohydrate offers a potentially superior crystalline form of anagrelide for storing or making pharmaceutical compositions.

Anagrelide hydrochloride monohydrate form II may be obtained by various processes. In essence, the procedures may be sorted into two groups:
a] Stirring a suspension of crystalline form I of anagrelide hydrochloride monohydrate in an alcoholic, ester, or ketone solvent (solvent-mediated polymorphic transition).
   The alcoholic solvent is an aliphatic C₁-C₄ alcohol, preferably chosen from ethanol, 2-propanol, or a mixture thereof. An example of a suitable ester is ethyl acetate. An example of a suitable ketone is acetone. The temperature of stirring is preferably at an ambient temperature; typically, the temperature of stirring is lower than 70°C, preferably from 15 to 35°C. The stirring time is typically at least 30 minutes, preferably of from 30 to 180 minutes.
   In an advantageous arrangement, a seeding crystal of form II may be used to facilitate the transformation.
b] Suspending anagrelide free base in a suitable solvent and contacting the suspension with a solution of hydrochloric acid in 2-propanol at an ambient temperature.

The anagrelide free base is preferably contacted with HCl by suspending anagrelide base in a solvent followed by contacting the formed suspension with an aqueous solution of HCl in 2-propanol under stirring. In one embodiment, an aqueous solution of HCl in 2-propanol is added to the anagrelide free base suspension under stirring. In another embodiment, the suspension of anagrelide base in a solvent is added to the aqueous solution of HCl in 2-propanol under stirring. The suitable solvent is selected from acetonitrile, ethanol, acetone, ethyl acetate, or a mixture thereof.

Anagrelide free base may be used in an isolated form, which advantageously is a solid form. Alternatively, a reaction mixture comprising anagrelide free base as a result of a chemical reaction may be used.

The temperature of stirring is preferably at an ambient temperature; typically, the temperature of stirring is from 15 to 35°C. The stirring time is typically at least several hours, preferably at least 2 hours.

In an advantageous arrangement, a seeding crystal of form II may be used to facilitate the transformation.

When anagrelide free base was suspended in 2-propanol and subsequently contacted with a solution of hydrochloric acid in 2-propanol, a third, solvated form III was identified. Alternatively, this form III is also obtained when a suspension of anagrelide hydrochloride monohydrate is stirred at reflux, filtered and vacuum-dried at ambient temperatures.

A characteristic XRPD pattern of form III is depicted in Figure 3. This polymorph is characterized by a distinct X-ray powder diffraction (XRPD) pattern comprising signals at diffraction angles of about 3.7°, 7.4°, 10.2°, 11.0°, 15.9°, 19.1°, 19.4°, 20.5°, 22.1°, 23.9°, 24.7°, 25.3°, 25.6°, 25.9°, 26.9°, 27.7°, 29.0°, 30.9°, 31.9°, 32.4°, 33.3°, and 34.4° degrees 2 theta (± 0.2 degrees 2 theta). This form III is unstable as it converts into form I or a mixture of forms I and II when exposed to air, and thus unsuitable for use in a pharmaceutical composition.

In any of the above processes, form II of anagrelide hydrochloride monohydrate may be advantageously isolated from the suspension thereof in a liquid by conventional techniques such as filtration or centrifugation, washed by a suitable liquid and dried. Air-drying of form II at ambient temperature does not cause any polymorphic change.

It is preferred that the formed crystals have an average particle size of 1,000 µm or smaller, preferably smaller than 250 µm, and more preferably smaller than 100 µm. The known particle size analysis methods can be used for determining the particle size, for example particle size measurement using light, like light-scattering methods, in particular Malvern Mastersizer.

The crystalline product of smaller particle size is, in general, easier to process in forming pharmaceutical compositions than that having a larger particle size. If such particle size is not obtainable by the process itself, conventional operations like sieving or milling can be performed to achieve a desired particle size (distribution).

The known processes for making crystalline anagrelide hydrochloride monohydrate are performed at reflux temperature. The processes of the present invention are performed at an ambient temperature; typically, the temperature of stirring is from 15 to 35°C, wherein anagrelide hydrochloride monohydrate form II is directly obtained.

Anagrelide hydrochloride monohydrate is unstable in water, and it converts into anagrelide free base within 30-180 minutes. This will also (partially) occur in a wet granulation process using water. It is apparent from the above that form II is a more stable and yet more soluble form of anagrelide hydrochloride monohydrate, at least in a suspension with ethanol, 2-propanol, or a mixture thereof. This represents an advantage, e.g. in a wet granulation processes using either of these solvents for making granulates, tablets or in pelletization processes. The physical stability of form II under contact with the granulation or pelletization liquid minimizes the danger of a polymorphic change of anagrelide hydrochloride monohydrate during the process, which could cause changes in properties of the resulting granulate, tablet or pellet. As no conversion or decomposition into the free base takes place, ethanol, 2-propanol, or a mixture thereof could also be suitable coating solvents.

Anagrelide hydrochloride monohydrate form II can be formulated into various pharmaceutical compositions with one or more pharmaceutically acceptable excipients. The pharmaceutical composition can be in a unit dosage form, such as a solid oral dosage form (i.e. a tablet or capsule), or a bulk precursor thereof, such as a pre-blended mixture ready for further blending/addition of ingredients, or a blend ready for tabletting or filling into capsules. Making a pharmaceutical composition from anagrelide hydrochloride monohydrate form II by a process which comprises combining it with said at least one pharmaceutically acceptable excipient is also disclosed herein. Also disclosed are pharmaceutical compositions, preferably solid oral pharmaceutical compositions, comprising a therapeutically effective amount of the form II of anagrelide hydrochloride monohydrate, in admixture with one or more pharmaceutically acceptable excipients. Pharmaceutically acceptable excipients may include, without limitation, carriers, diluents, fillers, binders, lubricants, disintegrants, glidants, colorants, pigments, taste masking agents, sweeteners, flavorants, plasticizers, etc. The proper excipient(s) are selected based in part on the dosage form, the intended mode of administration, the intended release rate, and manufacturing reliability.

Examples of common types of excipients include various polymers, waxes, calcium phosphates, sugars, etc. Polymers include cellulose and cellulose derivatives such as HPMC, hydroxypropyl cellulose, hydroxyethyl cellulose, microcrystalline cellulose, carboxymethylcellulose, sodium carboxymethylcellulose, calcium carboxymethylcellulose, and ethylcellulose; polyvinylpyrrolidones; polyethylenoxides; polyalkylene glycols such as polyethylene glycol and polypropylene glycol; and polyacrylic acids including their copolymers and crosslinked polymers thereof, e.g., Carbopol® (B.F. Goodrich), Eudragit® (Rohm), polycarbophil, and chitosan polymers. Waxes include carnauba wax, hydrogenated castor oil, glyceryl behenate, glycerylpalmito stearate, and saturated polyglycolyzed glycerate. Calcium phosphates include dibasic calcium phosphate, anhydrous dibasic calcium phosphate, and tribasic calcium phosphate. Sugars include simple sugars, such as lactose, maltose, mannitol, fructose, sorbitol, saccharose, xylitol, isomaltose, and glucose, as well as complex sugars (polysaccharides), such as maltodextrin, amylodextrin, starches, and modified starches.

Form II of anagrelide hydrochloride monohydrate may preferably be formulated into compositions for oral administration. The compositions for oral administration may be solid or liquid, such as in the form of an oral solution or suspension, oral capsule, or an oral tablet, and may exhibit immediate release or modified and/or extended release of the active substance from the composition.

The pharmaceutical compositions comprising form II of anagrelide hydrochloride monohydrate may be formulated, for instance, into conventional oral tablets. The tablets may be monolithic tablets, i.e. tablets that upon ingestion do not disintegrate into a plurality of smaller units from which the active ingredient is finally released, or may be disintegrable tablets. The tablets may be produced by any standard tabletting technique, e.g. by wet granulation, dry granulation, or direct compression. The wet granulation procedure comprises mixing anagrelide hydrochloride monohydrate and excipients with a granulation liquid and forming a solid granulated mass, which is then optionally dried, screening the granulate, mixing with remaining excipients, in particular with a lubricant, and compressing into a tablet. The dry granulation procedure typically comprises mixing the solid excipients (except lubricants), compacting the mixture in a compactor (e.g., a roller compactor), milling the compacted mass, screening the milled granules, mixing with a lubricant, and compressing the mixture into tablets. The direct compression procedure generally comprises mixing the solid excipients and compressing the uniform mixture into tablets.

In an alternative arrangement, the above produced wet or dry granulates or the direct mix of the solid excipients may be directly filled into capsules.

Anagrelide hydrochloride monohydrate form II may also be blended into compositions that are suitable for being formulated into pellets by known pelletization techniques. A plurality of pellets comprising a single dose of anagrelide may be encapsulated into capsules made from pharmaceutically acceptable material, such as hard gelatin. In another mode, a plurality of pellets may be compressed together with suitable binders and disintegrants to a disintegrable tablet that, upon ingestion, decomposes and releases the pellets. In yet another mode, the plurality of pellets may be filled into a sachet.

The relative amount of anagrelide hydrochloride monohydrate form II in the granulate, tablet or pellet mass is not particularly limited but typically may range from 0.1 to 10 wt%, such as 0.3 to 3.0 wt%.

The solid oral compositions and dosage forms comprising form II of anagrelide hydrochloride monohydrate are advantageously of an immediate release of anagrelide from the composition after ingestion. In some embodiments, they have the following release profile: more than 80% of the active is released in 30 minutes, preferably in 15 minutes, when measured by the paddle method of Ph.Eur. at 50 rpm in 0.01 M HCl.

Tablets or pellets may be coated by a suitable film coat, which may be a film coat (dissolvable in the stomach) or an "enteric coat" (not dissolvable in the stomach). The film coat may protect the tablet against the environment (light, air, moisture) during storage and handling. Any conventional film coat may be used. The enteric coat retards or inhibits the release of anagrelide in the stomach.

The unit dose in tablet form may comprise a single tablet but it may also comprise a divided tablet or several smaller tablets (minitablets) administered at the same time. The unit dose of pellets in capsule form may comprise a single capsule. Solutions for oral administration may be packed in a multidose package, the unit dose being packaged in a calibrated vessel.

The pharmaceutical dosage forms formulated from the compositions of the present invention may comprise a unit dose of anagrelide, i.e., a therapeutically effective amount of anagrelide hydrochloride monohydrate for a single dose administration. The amount of form II of anagrelide hydrochloride monohydrate, expressed as anagrelide base, in the daily unit dose may range from 0.1 to 15 mg, typically from 0.3 to 5.0 mg.

Form II of anagrelide hydrochloride monohydrate of the present invention or pharmaceutical compositions comprising it may be used in medicine, for instance in the treatment of (essential) thrombocythaemia. It may be used in monotherapy or in combination therapy.

The invention will be further described with reference to the following non-limiting examples.

### EXAMPLES

### Comparative example 1: Example 25 of US 3932407

0.16 g of anagrelide base was suspended in 7 ml acetonitrile (no clear solution at RT) and stirred at RT. To the suspension, 0.9 ml 1N HCl in diethyl ether was added. The suspension was stirred at RT for about 3 hours. The suspension was filtered over a P3-glass filter (reduced pressure). The solid was air-dried overnight at RT. An off-white solid with lumps was obtained.

XRPD showed all peaks characteristic of form I cf. Figure 1.

### Comparative example 2: based on Example 1 of US 5391737

Example 1 of US 5391737 does not give sufficient details as to the actual conduction of the experiment.

1.0 g of anagrelide base was suspended in 8.5 ml methanol, stirred and heated to reflux. No clear solution was obtained. To the hot suspension, 8.5 ml ethanol with 10-20% HCl was added and stirring at reflux was continued for about 15 minutes. The suspension slowly became thinner, but no clear solution was observed at all.

The suspension was allowed to cool to RT and filtered over a P3-glass filter (reduced pressure). The solid was washed with little ethanol, analysed with XRPD and air-dried overweekend at RT. An off-white powder with lumps was obtained.

XRPD showed unknown peaks. NMR indicated significant degradation into at least four impurities.

### Comparative example 3: Example 6 of WO 2009/087673

30 ml ethanol was heated up with an oilbath, while stirring. At 75°C, 1 gram of anagrelide base was added and the suspension was alllowed to reflux. Reflux and stirring was maintained for 10 minutes. To the suspension, 0.3 ml demi-water was added and reflux/stirring was continued for an additional 5 minutes. To the white, thick suspension, 1 ml concentrated HCl (37% aq) was added in a 3-minute period. As a result, the suspension became more yellowish and much thinner. After an additional 5 minutes at reflux, the suspension was cooled to 10-15°C taking about 15 minutes. The suspension was stirred for 15 min at 10-15°C. The suspension was filtered over a P3-glass filter (reduced pressure). The solid was washed with 2 ml ethanol and vacuum-dried at 30°C for about 4 hours. An off-white powder was obtained.

XRPD showed peaks pointing to a mixture of form I and form II cf. Figures 1 and 2.

### Example 1: Preparation of form II from anagrelide free base

0.5 g of anagrelide base was suspended in 20 ml ethanol (no clear solution at RT) and stirred at RT. To the suspension, 0.8 ml 5-6 N aqueous HCl in 2-propanol was added. The suspension was stirred at RT for about 2 hours. Then, the suspension was filtered over a P3-glass filter (reduced pressure). The solid was air-dried overnight at RT. An off-white powder with lumps was obtained.

XRPD showed all peaks characteristic of form II cf. Figure 2.

### Example 2: Preparation of form II from form I

∼0.5 g of anagrelide hydrochloride monohydrate form I was suspended in 5 ml ethanol and stirred at RT for about 2 days. The suspension was filtered over a P3-glass filter (reduced pressure). The solid was air-dried overweekend at RT. An off-white, fine powder was obtained.

XRPD showed all peaks characteristic of form II cf. Figure 2.

2.0 g of anagrelide hydrochloride monohydrate (a mixture of forms I and II), was suspended in 20 ml ethanol. The mixture was stirred overnight at RT in a closed flask. The suspension was filtered over a P3-glass filter (reduced pressure). The solid was washed with ethanol and air-dried overweekend at RT. An off-white to pale-beige, fine powder was obtained.

XRPD showed all peaks characteristic of form II cf. Figure 2.

### Example 3: Preparation of form III

15.0 g anagrelide hydrochloride monohydrate was suspended in 150 ml 2-propanol, heated to reflux and stirred at reflux for 2 hours. Then, 1 ml 5-6 N aqueous HCl in 2-propanol was added and the suspension was cooled down to luke-warm. The suspension was filtered over a P3-glass filter (reduced pressure). The solid was washed with 2-propanol and air-dried at RT. An off-white to yellowish, fine powder was obtained.

XRPD showed all peaks characteristic of form III cf. Figure 3.

### Stability data of forms I and II

### Stability of form I

0.5-15 g of anagrelide hydrochloride monohydrate form I was suspended in 5-25 ml of solvent and stirred at RT for 0.5-24 hours. The suspension was filtered over a P3-glass filter (reduced pressure), washed with solvent and dried. Details and results are provided in the table below.

| **Solvent** | **Stirring time** | **Stirring temperature** | **XRPD** |
|---|---|---|---|
| Demi-water | 30 min | RT* | Anagrelide base |
| | 180 min | RT | Anagrelide base |
| Ethanol | 30 min | RT | Form II |
| | 16 hours | RT | Form II |
| 2-propanol | 16 hours | RT | Form I + II |
| Acetone | 2 days | RT | Form II |
| Ethyl acetate | 2 days | RT | Form II |

| | | | |
|---|---|---|---|
| * RT "means room temperature" (between 15-25°C, typically between 20-21°C). | | | |

### Stability of form II

2.0 g of anagrelide hydrochloride monohydrate form II was suspended in 20 ml solvent. The mixture was stirred at RT in a closed flask. At certain time points, a small amount of the suspension was filtered over a P3-glass filter (reduced pressure). The solids were air-dried overnight at RT. Details and results are provided in the table below.

| **Solvent** | **Stirring time** | **Stirring temperature** | **XRPD** |
|---|---|---|---|
| Demi-water | 30 min | RT* | Form II + anagrelide base |
| | 60 min | RT | Form II + anagrelide base |
| | 120 min | RT | Form II + anagrelide base |
| | 180 min | RT | Anagrelide base |
| Ethanol | 30 min | RT | Form II |
| | 60 min | RT | Form II |
| | 120 min | RT | Form II |
| | overnight | RT | Form II |
| | 3 hours | reflux | Form I |
| 2-propanol | 30 min | RT | Form II |
| | 60 min | RT | Form II |
| | 120 min | RT | Form II |
| | overnight | RT | Form II |

| | | | |
|---|---|---|---|
| * RT "means room temperature" (between 15-25°C, typically between 20-21°C). | | | |

### Conversion study in ethanol

0.25 g of anagrelide hydrochloride monohydrate (a mixture of forms I and II) was suspended in 5 ml of ethanol and stirred at a specified stirring temperature for 22-24 hours. The suspension was filtered over a P3-glass filter (reduced pressure), washed with solvent and dried. Experimental details and results are provided below.

| **Stirring temperature (°C)** | **Crystal form (XRPD)** |
|---|---|
| 30 | II |
| 35 | II |
| 40 | II |
| 45 | II |
| 50 | II |
| 55 | II |
| 60 | II |
| 65 | II |
| 67.5* | II |
| 70* | II |
| 72.5* | I + II |
| 75* | mostly I, some II left |

| | |
|---|---|
| * Stirring time 2 hours only. | |

Form II is stable at temperatures below 70°C, but is not stable at reflux. The transition point lies around 72.5°C.

Thus, at temperatures below 72.5°C form II will crystallise, whereas at temperatures above 72.5°C form I will preferentially be formed.

In the above examples, the XRPD patterns were recorded on a Bruker-AXS D8 vario (Θ/2Θ geometry, reflection mode, Vantec PSD detector) operating at the following settings:

| | |
|---|---|
| Start angle (2*θ*): | 2.0° |
| End angle (2*θ*): | 35.0° |
| Scan step width: | 0.02° |
| Scan step time: | between 0.7-5.5 seconds |
| Radiation type: | Cu |
| Radiation wavelengths: | 1.54060 Å (Kα₁), primary monochromator used |
| Exit slit: | 6.0 mm |
| Focus slit: | 0.2 mm |
| Divergence slit: | Variable (V20) |
| Antiscatter slit: | 11.8 mm |
| Receiving slit: | 20.7 mm |

### Solubility experiments

The instant solubility of anagrelide hydrochloride monohydrate form II, form I and free base was determined in USP acetate buffer pH 4.5, USP phosphate buffer pH 6.8, Milli-Q water (herein below "MQ") and 0.1 M HCl. About 30 mg of the drug substance was transferred into a clear 50 ml sample jar and a stirring bar was added. Subsequently 30 ml of test medium was added; the vial was closed and placed on a magnetic stirrer. Under constant stirring at room temperature samples of 2 ml were taken at t=0, 10, 20, 30, 40, 50, and 60 minutes. The samples were immediately filtered through a 0.45 µm syringe disk filter (first ml was discarded) and analyzed at once with the HPLC-UV method described in Table 1. The solubility was calculated in mg/ml anagrelide free base against an external standard (AGL.hcl.mhy.120310) dissolved in acetonitrile/milli-Q (ACN/MQ) (50:50).

**Table 1 HPLC-UV parameters used for instant solubility testing**

| | |
|---|---|
| **Column:** | Xbridge Shield RP18, 50x4.6mm, 3.5µm (Waters) |
| **Column temperature:** | 30°C |
| **Auto sampler temp.:** | Room temperature |
| **Flow:** | 1.2 ml/min, isocratic |
| **Detection:** | UV, 254 nm |
| **Injection volume:** | 3 µl |
| **Mobile phase:** | 50 mM KH₂PO₄ to pH=2.5 / ACN (75:25) |
| **Run time:** | 3.5 min. |
| **Next injection delay:** | 0 min. |

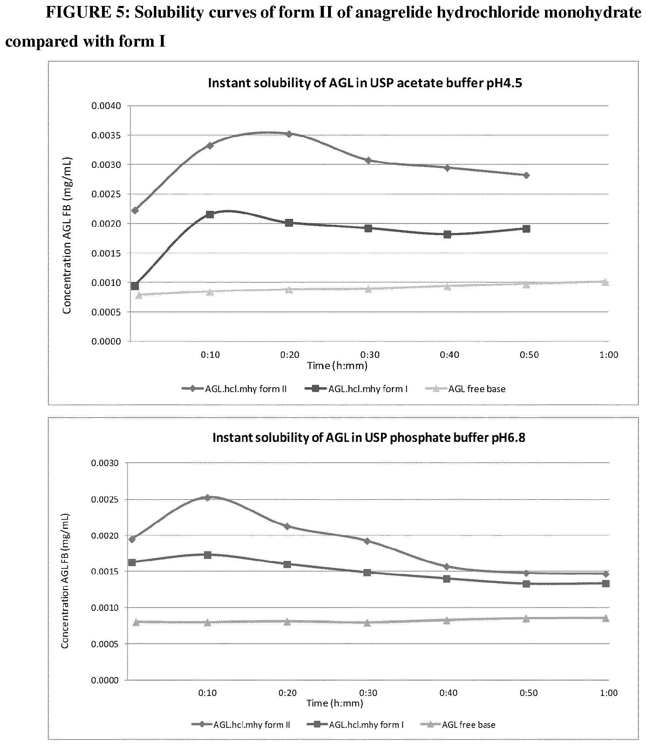

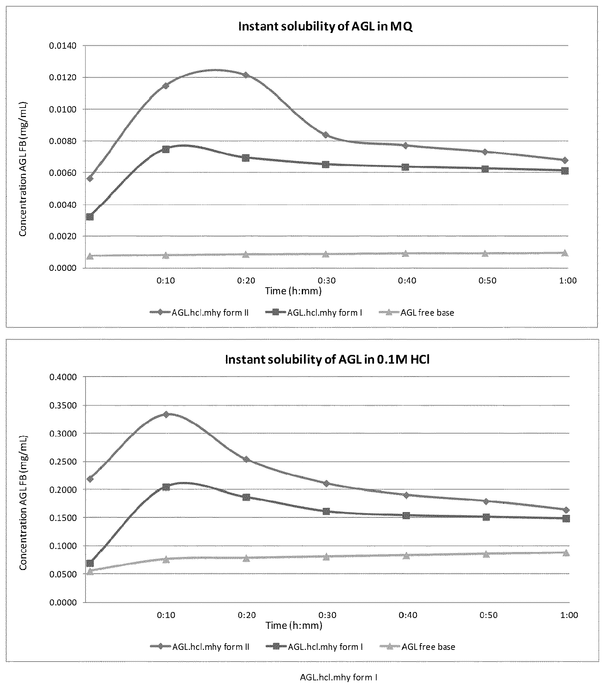

The results are shown in Figure 5. In general, the instant solubility curves of form II are higher compared to form I indicating a higher instant solubility.

## Claims

1. Crystalline form II of anagrelide hydrochloride monohydrate **characterized by** an XRPD pattern that includes the following peaks at 2θ: 14.2, 15.3, 16.3, 18.7, 19.5, 22.4, 23.1, 23.5, 23.7, 24.2, 26.6, and 33.6 ± 0.2 degrees, when measured with CuKα1 radiation (λ = 1.54060 Å) having a polymorphic purity of at least 90%.

2. A process for making the crystalline form II of anagrelide hydrochloride monohydrate according to claim 1 from crystalline form I of anagrelide hydrochloride monohydrate comprising:
a) Suspending crystalline form I in ethanol, 2-propanol or a mixture thereof or ketone solvent;
b) Stirring at an ambient temperature between 15 and 35°C for at least 30 minutes, preferably of from 30 to 180 minutes; and
c) Isolating the obtained crystals of form II.

3. The process according to claim 2, wherein the suspension of step a) is seeded with crystals of form II according to claim 1.

4. A process for making the crystalline form II of anagrelide hydrochloride monohydrate according to claim 1 comprising:
a) Suspending anagrelide free base in a solvent at an ambient temperature between 15 and 35°C;
b) Contacting the suspension of step a) with a solution of hydrochloric acid in 2-propanol at an ambient temperature between 15 and 35°C;
c) Stirring at an ambient temperature between 15 and 35°C for several hours, preferably more than 2 hours; and
d) Isolating the obtained crystals of form II according to claim 1;
wherein the solvent in step a) is selected from acetonitrile, ethanol, acetone, ethyl acetate, or a mixture thereof.

5. The process according to claim 4, wherein the stirring of step c) is seeded with crystals of form II according to claim 1.

6. Crystalline form II of anagrelide hydrochloride monohydrate according to claim 1 formulated into a tablet, capsule, granulate or pellet comprising a pharmaceutically acceptable excipient wherein the relative amount of anagrelide hydrochloride monohydrate form II in the tablet, capsule, granulate or pellet mass range from 0.1 to 10 wt%.

## Patentansprüche

1. Kristalline Form II von Anagrelidhydrochloridmonohydrat, **gekennzeichnet durch** ein XRPD-Muster, das die folgenden Peaks bei 2θ aufweist: 14.2, 15.3, 16.3, 18.7, 19.5, 22.4, 23.1, 23.5, 23.7, 24.2, 26.6, und 33.6 ± 0.2 Grad, gemessen mit CuKα1-Strahlung (λ = 1.54060 Å) mit einer polymorphen Reinheit von mindestens 90%.

2. Verfahren zur Herstellung der kristallinen Form II von Anagrelidhydrochloridmonohydrat nach Anspruch 1 aus der kristallinen Form I von Anagrelidhydrochloridmonohydrat, umfassend:
a) Suspendieren der kristallinen Form I in Ethanol, 2-Propanol oder einem Gemisch davon oder Ketonlösungsmittel;
b) Rühren bei einer Umgebungstemperatur zwischen 15 und 35°C für mindestens 30 Minuten, vorzugsweise 30 bis 180 Minuten; und
c) Isolieren der erhaltenen Kristalle der Form II.

3. Verfahren nach Anspruch 2, wobei die Suspension von Schritt a) mit Kristallen der Form II nach Anspruch 1 beimpft wird.

4. Verfahren zur Herstellung der kristallinen Form II von Anagrelidhydrochloridmonohydrat nach Anspruch 1, umfassend:
a) Suspendieren einer freien Base von Anagrelid in einem Lösungsmittel bei einer Umgebungstemperatur zwischen 15 und 35°C;
b) Inkontaktbringen der Suspension von Schritt a) mit einer Lösung von Chlorwasserstoffsäure in 2-Propanol bei einer Umgebungstemperatur zwischen 15 und 35°C;
c) Rühren bei einer Umgebungstemperatur zwischen 15 und 35°C für mehrere Stunden, vorzugsweise mehr als 2 Stunden; und
d) Isolieren der erhaltenen Kristalle der Form II nach Anspruch 1;
wobei das Lösungsmittel in Schritt a) ausgewählt ist aus Acetonitril, Ethanol, Aceton, Ethylacetat oder einem Gemisch davon.

5. Verfahren nach Anspruch 4, wobei das Rühren von Schritt c) mit Kristallen der Form II nach Anspruch 1 beimpft wird.

6. Kristalline Form II von Anagrelidhydrochloridmonohydrat nach Anspruch 1, formuliert zu einer Tablette, einer Kapsel, einem Granulat oder einem Pellet, die/das einen pharmazeutisch annehmbaren Trägerstoff umfasst, wobei die relative Menge an Anagrelidhydrochloridmonohydrat der Form II in der Tabletten-, Kapsel-, Granulat- oder Pellet-Masse von 0.1 bis 10 Gew.-% beträgt.

## Revendications

1. Forme cristalline II de chlorhydrate d'anagrélide monohydraté **caractérisée par** un diagramme XRPD qui comporte les pics suivants à 2θ : 14.2 ; 15.3 ; 16.3 ; 18.7 ; 19.5 ; 22.4 ; 23.1 ; 23.5 ; 23.7 ; 24.2 ; 26.6 et 33.6 ± 0.2 degrés, lors d'une mesure avec un rayonnement CuKα1 (λ = 1.54060 Å) présentant une pureté polymorphe d'au moins 90 %.

2. Procédé de fabrication de la forme cristalline II de chlorhydrate d'anagrélide monohydraté selon la revendication 1 à partir de la forme cristalline I de chlorhydrate d'anagrélide monohydraté comprenant :
a) la mise en suspension de la forme cristalline I dans l'éthanol, le 2-propanol ou un mélange de ceux-ci ou un solvant à base de cétone ;
b) l'agitation à une température ambiante entre 15 et 35 °C pendant au moins 30 minutes, de préférence de 30 à 180 minutes ; et
c) l'isolement des cristaux obtenus de forme II.

3. Procédé selon la revendication 2, dans lequel la suspension de l'étape a) est ensemencée avec des cristaux de forme II selon la revendication 1.

4. Procédé de fabrication de la forme cristalline II de chlorhydrate d'anagrélide monohydraté selon la revendication 1, comprenant :
a) la mise en suspension d'une base libre d'anagrélide dans un solvant à une température ambiante entre 15 et 35 °C ;
b) la mise en contact de la suspension de l'étape a) avec une solution d'acide chlorhydrique dans du 2-propanol à une température ambiante entre 15 et 35 °C ;
c) l'agitation à une température ambiante entre 15 et 35 °C pendant plusieurs heures, de préférence plus de 2 heures ; et
d) l'isolement des cristaux obtenus de forme II selon la revendication 1 ;
dans lequel le solvant à l'étape a) est choisi parmi l'acétonitrile, l'éthanol, l'acétone, l'acétate d'éthyle ou un mélange de ceux-ci.

5. Procédé selon la revendication 4, dans lequel l'agitation de l'étape c) est ensemencée avec des cristaux de forme II selon la revendication 1.

6. Forme cristalline II de chlorhydrate d'anagrélide monohydraté selon la revendication 1 formulée en un comprimé, une capsule, un granulé ou une pastille comprenant un excipient pharmaceutiquement acceptable dans laquelle la quantité relative de forme II de chlorhydrate d'anagrélide monohydraté dans la masse du comprimé, de la capsule, du granulé ou de la pastille est dans la plage de 0.1 à 10 % en poids.
